# FASCICULE DE BREVET EUROPEEN

(11) **EP 0 810 631 B1**
(45) Date de publication et mention de la délivrance du brevet: **04.09.2002**
(21) Numéro de dépôt: 97401150.4
(22) Date de dépôt: 26.05.1997
(51) Int. Cl.: H01J 47/02

(54) **Dispositif d'imagerie radiographique à haute résolution**
Hochauflösende radiografische Bilderzeugungsvorrichtung
High resolution radiographic imaging device

(30) Priorité: 29.05.1996 FR 9606600
(43) Date de publication de la demande: 03.12.1997
(73) Titulaire: BIOSPACE INSTRUMENTS, 75011 Paris (FR)
(72) Inventeur: Charpak, Georges, 75005 Paris (FR)
(74) Mandataire: Fréchède, Michel

(56) Documents cités:
- EP-A- 0 678 896
- FR-A- 2 680 010
- FR-A- 2 739 941
- US-A- 5 032 729
- US-A- 5 308 987

## Description

L'invention concerne un dispositif d'imagerie radiographique à haute résolution.

D'une manière générale, les dispositifs d'imagerie radiographique actuels sont soumis à deux contraintes essentielles, l'accroissement de la résolution des images obtenues, et la réduction des doses de rayonnement appliquées au sujet à observer, notamment par la réduction du temps d'exposition au rayonnement ionisant de ce dernier.

En ce qui concerne l'accroissement de la résolution des images, un tel objectif apparaît, avant tout, lié à la finesse de détection du rayonnement ionisant illuminant le sujet observé, à la finesse spatiale des motifs physiques engendrés par ce processus de détection, et finalement à la finesse géométrique des éléments détecteurs permettant la mise en évidence de ces motifs physiques.

En outre, la mise en oeuvre d'éléments détecteurs de très haute résolution, et donc de dimensions très faibles, et à grand gain d'amplification, est susceptible de permettre une distribution appropriée d'une pluralité de ce type d'éléments détecteurs sur un domaine d'observation spécifique et de réduire en conséquence le temps d'illumination nécessaire à l'observation de ce domaine à celui nécessaire à l'observation d'un domaine élémentaire correspondant à celui d'un élément détecteur, ce qui permet, en conséquence, de réduire de manière significative les doses de rayonnement appliquées au sujet à observer.

Un dispositif d'imagerie médicale en rayonnement ionisant X ou gamma à faible dose a été décrit par la demande de brevet européen 0 678 896 déposée par Monsieur Georges CHARPAK. Ce dispositif est basé sur l'association, dans une enceinte à gaz, d'une chambre de dérive et d'une chambre à fils comprenant une grille de multiplication proportionnelle (13) des électrons, la détection des électrons multipliés étant assurée par une électrode (14) formant une électrode de cathode. Ce dispositif donne satisfaction mais présente les limitations, notamment de gain, inhérentes aux grilles à multiplication proportionnelle.

La présente invention a pour objet la mise en oeuvre d'un dispositif d'imagerie radiographique à haute résolution permettant de satisfaire aux contraintes précitées.

Un autre objet de la présente invention est la mise en oeuvre d'un dispositif d'imagerie radiographique dans lequel la résolution d'image est meilleure que 100 µm.

Un autre objet de la présente invention est la mise en oeuvre d'un dispositif d'imagerie radiographique dans lequel le temps d'exposition d'un sujet peut être réduit par un facteur 10 par rapport aux dispositifs de l'art antérieur.

Le dispositif d'imagerie radiographique en rayonnement ionisant à haute résolution, objet de la présente invention, comporte une source de rayonnement ionisant selon un faisceau divergent, une fente longitudinale formant diaphragme permettant de délivrer un faisceau d'illumination en nappe, distribué sensiblement dans un plan contenant la fente longitudinale, et un module de détection d'un faisceau transmis par un sujet à observer, illuminé par le faisceau d'illumination en nappe, comprenant une enceinte à gaz munie d'une fenêtre d'admission du faisceau d'illumination.
Il est remarquable en ce que le module de détection comporte au moins un détecteur de particules ionisantes comprenant au moins, dans l'enceinte à gaz, une première, une deuxième, une troisième électrode plane, chaque électrode plane étant placée parallèlement chacune à chacune pour former d'une part entre les première et deuxième électrodes, un espace de conversion du faisceau d'illumination en électrons et, d'autre part, entre les deuxième et troisième électrodes, un espace d'amplification par multiplication de ces électrons. La fenêtre d'admission est placée au niveau de l'espace de conversion pour assurer l'admission du faisceau d'illumination dans l'espace de conversion et l'espace d'amplification présente une longueur de multiplication, distance séparant la deuxième et la troisième électrode, inférieure à 200 µm. Le détecteur comporte en outre un circuit de polarisation permettant de porter la première électrode à un premier potentiel électrique, la deuxième électrode à un deuxième potentiel électrique supérieur au premier potentiel électrique, et la troisième électrode à un troisième potentiel électrique supérieur au deuxième potentiel électrique. La deuxième électrode percée de trous forme une cathode permettant la transmission des électrons engendrés dans l'espace de conversion vers l'espace d'amplification, et la troisième électrode forme une anode constituée par une pluralité d'anodes élémentaires isolées électriquement. Les potentiels électriques appliqués à la première, à la deuxième et à la troisième électrode permettent d'engendrer un premier champ électrique E₁ dans l'espace de conversion et un deuxième champ électrique E₂ sensiblement parallèle dans l'espace d'amplification, ces champs électriques E₁, E₂ étant dans un rapport d'amplitude ρ=E₂/E₁ supérieur à 10, chacun de ces champs électriques E₁ et E₂ étant sensiblement orthogonal auxdites électrodes, pour assurer la multiplication de ces électrons dans l'espace d'amplification par phénomène d'avalanche, confiné à la longueur d'amplification, et engendrer, au voisinage de la troisième électrode formant anode, des électrons multipliés. Des circuits de détection et de comptage des électrons multipliés sont couplés aux anodes élémentaires formant l'anode.

Le dispositif d'imagerie radiographique à haute résolution trouve application, tant relativement à l'imagerie radiographique industrielle, notamment à la cristallographie et la résistance des matériaux, qu'à l'imagerie radiographique médicale.

Il sera mieux compris à la lecture de la description ci-après et à l'observation des dessins dans lesquels :
- les figures la et 1b représentent une vue en coupe longitudinale d'un dispositif d'imagerie radiographique à haute résolution, objet de la présente invention ;
- les figures 1c et 1d représentent une vue en coupe selon le plan de coupe AA transversal de la figure la, relativement à une première et une deuxième variante de réalisation du dispositif d'imagerie radiographique à haute résolution, objet de la présente invention ;
- la figure 2a représente un détail de réalisation de circuits de détection spécifiques mis en oeuvre par le dispositif d'imagerie radiographique à haute résolution selon l'invention ;
- la figure 2b représente un détail de réalisation avantageux de la grille formant cathode d'un élément de détection conforme à l'objet de la présente invention ;
- les figures 3a et 3b représentent un mode de réalisation avantageux d'un dispositif d'imagerie radiographique à haute résolution selon l'invention, dans lequel une structure multicanaux est placée dans l'espace de conversion du rayonnement ionisant d'un élément détecteur mis en oeuvre par ce dispositif, et un détail de réalisation de cette structure multicanaux ;
- les figures 4a à 4d représentent une variante de réalisation du dispositif d'imagerie radiographique haute résolution, objet de l'invention, dans lequel un circuit à couplage capacitif est utilisé pour assurer la détection des électrons engendrés par le rayonnement ionisant ;
- les figures 5a et 5b représentent une autre variante de réalisation du dispositif d'imagerie radiographique haute résolution, objet de l'invention, dans lequel la détection des électrons engendrés par le rayonnement ionisant est réalisée grâce à la création de phénomènes d'avalanches lumineuses ;
- la figure 5c représente un dispositif d'imagerie radiographique à haute résolution conforme à l'objet de la présente invention en imagerie β ;
- les figures 6a et 6b représentent un dispositif d'imagerie radiographique à haute résolution conforme à l'objet de la présente invention, dans lequel une pluralité d'éléments détecteurs élémentaires sont agencés par superposition, de façon à permettre une analyse tridimensionnelle d'un sujet donné pour un faisceau d'illumination d'angle solide déterminé.

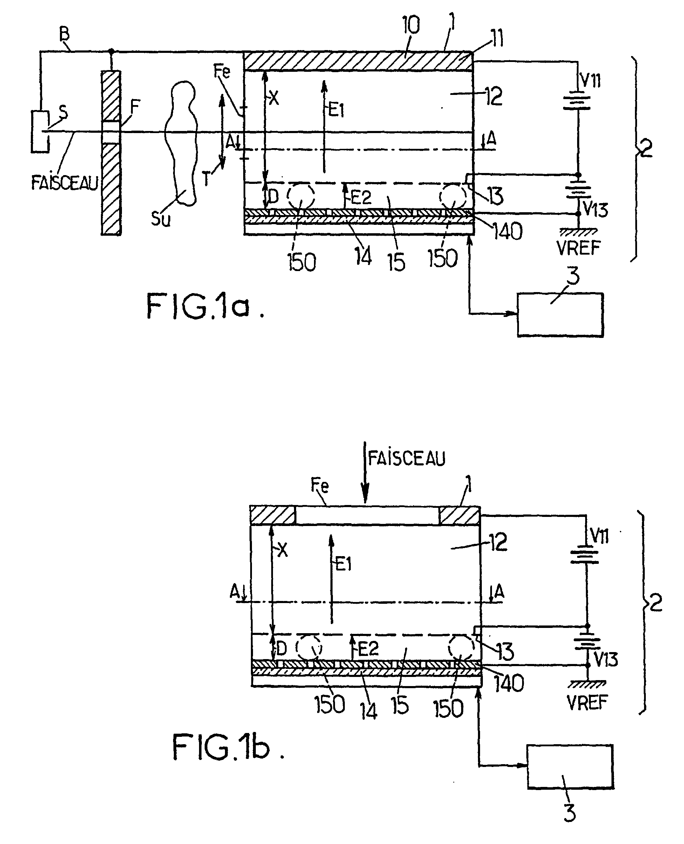

Une description plus détaillée d'un dispositif d'imagerie radiographique en rayonnement ionisant à haute résolution, conforme à l'objet de la présente invention, sera maintenant donnée en liaison avec les figures 1a et 1b.

La figure la précitée est une vue en coupe selon un plan de symétrie longitudinal de ce dispositif, ce plan de symétrie longitudinal étant, par définition, le plan de la feuille dans laquelle la figure 1a est représentée.

Ainsi qu'on l'observera sur la figure précitée, le dispositif selon l'invention comprend une source de rayonnement ionisant, notée S, délivrant un rayonnement ionisant selon un faisceau divergent, et une fente longitudinale, notée F, formant un diaphragme, permettant de délivrer un faisceau d'illumination. De manière classique, ce faisceau d'illumination est un faisceau en nappe de rayons X ou γ (gamma) distribué sensiblement dans le plan contenant la fente longitudinale F, c'est-à-dire dans un plan perpendiculaire au plan de la feuille sur laquelle est représentée la figure 1a. Le faisceau d'illumination précité permet d'illuminer un sujet SU à observer, ce faisceau d'illumination, après absorption sélective en fonction des zones de densité du sujet à observer SU, étant reçu par un module de détection, noté 1 sur la figure la précitée.

Selon un aspect particulièrement avantageux du dispositif objet de la présente invention, on indique que le module de détection 1 comporte au moins un détecteur de particules ionisantes comprenant une enceinte à gaz 10, munie d'une fenêtre d'admission du faisceau d'illumination.

L'enceinte à gaz 10 est une enceinte de type classique présentant une fenêtre d'admission du faisceau d'illumination en nappe, fenêtre notée Fe, et d'éléments d'admission d'un gaz de remplissage, ces éléments d'admission n'étant pas représentés aux dessins afin de ne pas surcharger ces derniers. Le gaz admis dans l'enceinte à gaz peut, de manière classique, être admis en légère surpression par rapport à la pression atmosphérique, ou, au contraire, soit à une pression relativement élevée, soit à très basse pression, voisine de quelques Torr, en fonction des conditions d'utilisation du dispositif d'imagerie radiographique, objet de l'invention.

Ainsi qu'il apparaît en outre en figures 1a et 1b, l'enceinte à gaz 10 comporte une première électrode, notée 11, une deuxième électrode, notée 13, et une troisième électrode, notée 14, ces électrodes étant planes et placées parallèlement chacune à chacune pour former un espace de conversion, noté 12, du faisceau d'illumination en électrons, et un espace d'amplification par multiplication de ces électrons, cet espace d'amplification étant noté 15.

La fenêtre d'admission Fe est placée au niveau de l'espace de conversion 12 et permet d'assurer l'admission du faisceau d'illumination dans l'espace de conversion 12 parallèlement à la première, 11, et à la deuxième électrode, 13, dans le cas du mode de réalisation de la figure 1a.

Dans le mode de réalisation de la figure 1a, la fenêtre d'admission Fe est ainsi placée latéralement sur l'enceinte à gaz 10, de façon à permettre l'admission du faisceau d'illumination parallèlement aux électrodes de cathode 13 et d'anode 14.

Au contraire, dans le mode de réalisation de la figure 1b, la fenêtre d'admission Fe est placée sur la face de l'enceinte à gaz située en vis-à-vis de la deuxième électrode de cathode 13 de façon à permettre une admission frontale du faisceau d'illumination vis-à-vis des électrodes.

On comprend bien sur que l'ensemble constitué par la source S, la fente F matérialisée par un diaphragme en plomb par exemple, et le module détecteur 1 peut être rendu solidaire par l'intermédiaire d'un bâti B, cet ensemble pouvant être manoeuvré en translation, et, le cas échéant, en rotation, pour assurer une analyse du sujet SU appropriée. La réalisation mécanique de l'ensemble ne sera pas décrite en détail car les éléments nécessaires à cette réalisation sont connus de l'homme de l'art. En outre la source S et le module détecteur 1 peuvent être fixes, le sujet SU étant mis en mouvement selon un déplacement approprié.

Selon une caractéristique particulièrement avantageuse du dispositif, objet de la présente invention, on indique que l'espace d'amplification 15 présente une longueur de multiplication, c'est-à-dire une distance D séparant la deuxième, 13, et la troisième électrode 14, inférieure à 200 µm.

Selon une autre caractéristique particulièrement remarquable du dispositif, objet de la présente invention, on indique que celui-ci comprend un circuit de polarisation, noté 2, permettant de porter la première électrode 11 à un premier potentiel électrique, la deuxième électrode 13 à un deuxième potentiel électrique supérieur au premier, et la troisième électrode 14 à un troisième potentiel électrique supérieur au deuxième potentiel électrique.

Ainsi que représenté sur la figure la, le circuit de polarisation précité peut comprendre une source d'alimentation continue, notée V₁₃, dont l'électrode positive est reliée à la tension de référence V_{REF}, ainsi qu'à la troisième électrode 14, et donc l'électrode négative est reliée à la deuxième électrode 13. En outre, ce circuit de polarisation comprend une autre source de tension, notée V11, dont l'électrode positive est reliée à l'électrode négative de la source V₁₃ et dont l'électrode négative est reliée à la première électrode 11.

On comprend ainsi que la deuxième électrode 13 forme une électrode de cathode, alors que la troisième électrode 14 forme une électrode d'anode.

La distance X séparant la première, 12, et la deuxième électrode 13 est très supérieure à la distance D séparant la deuxième électrode 13 et la troisième électrode 14. A titre d'exemple non limitatif, pour des particules chargées en hautes énergies et pour une admission frontale du faisceau d'illumination, on indique que pour une distance D = 100 µm, la distance X peut être prise égale à 3 mm. Toutefois, le faible volume de l'espace d'amplification 15 permet de choisir une distance X, distance entre la première 11 et la deuxième électrode 13, égale à une valeur très voisine de la dimension, dans cette même direction, de la fente Fe dans le cas de la figure 1a. Ainsi la distance X peut être prise égale à 1 mm en particulier dans le cas où le gaz remplissant l'enceinte à gaz est le xénon. Dans un tel cas, lorsque le faisceau d'illumination est un faisceau de rayons X, d'énergie 50 keV par exemple, pour un photon X il apparaît dans l'espace de conversion 12 un photoélectron d'énergie 15 keV et un électron secondaire issu d'un rayonnement de fluorescence X dans le gaz d'énergie voisine de 30 keV, le photoélectron et l'électron secondaire ne pouvant être séparés dans les détecteurs classiques. L'apparition de deux signaux dans ces détecteurs, conduit à rejeter l'événement. La réduction de la distance X à une valeur de l'ordre du millimètre a pour effet de réduire très fortement la probabilité de capture du photon de fluorescence X, ce qui permet donc de supprimer l'apparition d'un signal double.

En outre, et selon un aspect particulièrement remarquable du dispositif, objet de la présente invention, les sources de tension V₁₃ et V₁₁ peuvent être choisies de façon que les potentiels électriques appliqués à la première, 11, à la deuxième, 12, et à la troisième électrode, permettent d'engendrer un premier, E₁, et un deuxième, E₂, champ électrique, ces champs électriques étant constitués par des vecteurs champs électriques parallèles dans l'espace de conversion 12 ainsi que dans l'espace d'amplification 13. Ces champs électriques étant orthogonaux aux électrodes précitées et dans un rapport d'amplitude ρ = E₂ / E₁ > 10.

Dans un mode de réalisation non limitatif, on indique que le rapport ρ est pris égal à 50.

En outre, on indique que la deuxième électrode est formée par une grille percée de trous et permet la transmission des électrons engendrés dans l'espace de conversion 12 vers l'espace d'amplification 15, ce qui, compte tenu des valeurs d'amplitude de champ et de la distance d'amplification D, permet d'assurer la multiplication de ces électrons dans l'espace d'amplification 15 par un phénomène d'avalanche confiné à la longueur d'amplification D précitée, pour engendrer, au voisinage de la troisième électrode 14 formant une anode, des électrons multipliés.

D'une manière avantageuse, on indique que la troisième électrode 14 formant anode est formée d'anodes élémentaires dans les conditions qui seront décrites ultérieurement dans la description.

Enfin, un circuit de détection 3 est prévu, lequel permet d'assurer la détection et le comptage des électrons multipliés, ce circuit de détection et de comptage 3 étant couplé aux anodes élémentaires formant l'anode.

Dans un mode de réalisation préférentiel, un indique que les sources de tension V₁₃ et V₁₁ sont choisies de façon à engendrer un champ E₂ dans l'espace d'amplification 15, d'amplitude 100 kV/cm, pour un remplissage à la pression atmosphérique alors que le champ électrique E₁ engendré dans l'espace de conversion 12 présente une amplitude égale à 1 kV/cm, ce qui permet d'obtenir un rapport ρ = 100.

La configuration de l'élément détecteur 1 tel que représenté en figure 1a, c'est-à-dire les valeurs de dimension D et X précitées ainsi que la valeur de rapport de champ engendré entre l'espace d'amplification 15 et l'espace de conversion 12, permet un fonctionnement particulièrement remarquable en ce que les électrons créés dans l'espace de conversion 12, remplis de gaz, sont transmis par l'électrode de cathode 13 dans l'espace d'amplification 15, dans lequel, en raison de la très forte valeur du champ électrique E₂, ces électrons sont soumis à un phénomène d'avalanche, lequel bien entendu est confiné sur la distance d'avalanche D, laquelle est très faible. Pour cette raison, le front d'avalanche, c'est-à-dire la dimension dans une direction perpendiculaire à la direction du champ électrique E₂, est également très faible, au plus égale à la distance de multiplication D/10 pour un gain de 1000. En effet, un électron qui pénètre dans l'espace d'amplification 15 est multiplié par des chocs successifs. Pour un gain d'amplification de l'ordre de 1000, alors que 1000 # 2ⁿ, soit n=10, et le libre parcours moyen de multiplication est λ = D/10. La taille du front d'avalanche est donc de l'ordre de λ, soit D/10. Ainsi, la dimension réelle des motifs d'avalanche engendrés par la multiplication de chaque électron dans l'espace d'amplification 15, atteint au plus, au voisinage de l'électrode 14, une valeur de l'ordre de celle du libre parcours λ de l'ordre de D/10, ce qui permet d'atteindre une valeur de résolution correspondant à cette dimension grâce à la mise en oeuvre d'anodes élémentaires de dimension correspondante, ainsi qu'il sera décrit ci-après dans la description.

En outre, on indique également que, en raison de la proximité due à la faible valeur de la distance de multiplication D entre la deuxième électrode 13 de cathode et la troisième électrode 14 d'anode, les ions positifs créés du fait des phénomènes d'avalanche, ions positifs susceptibles de créer une charge d'espace au niveau de l'anode 14, sont absorbés en un temps très court, de l'ordre de 100 ns, ce qui autorise un taux de comptage très élevé, supérieur à environ 10⁶ coups/s/mm². Les taux de comptage autorisés sont donc de 100 à 1000 fois plus importants que ceux qui sont susceptibles d'être atteints avec les chambres à fils classiques.

On indique que, au contraire, dans les détecteurs de type classique, tels que les chambres à fils, le temps de collecte des ions positifs peut atteindre quelques dizaines de µs, en conséquence de quoi les taux de comptage sont limités à des valeurs 100 fois plus faibles.

Pour une description plus détaillée de l'élément détecteur 1 précité, on pourra utilement se reporter à la demande de brevet français FR-A 2 739 941 au nom de Monsieur Georges CHARPAK et du COMMISSARIAT A L'ENERGIE ATOMIQUE.

D'une manière particulièrement avantageuse, ainsi que représenté en figure 1c, les anodes élémentaires constitutives de la troisième électrode formant l'anode 14 peuvent être formées par des éléments électriquement conducteurs, notés 140 sur la figure 1c précitée, ces éléments conducteurs présentant un axe de symétrie longitudinale noté YY, sur cette figure. Ainsi qu'on l'observera sur la figure précitée, l'axe de symétrie longitudinale YY de chaque élément électriquement conducteur 140 est orienté de manière à converger vers la source de rayonnement ionisant S, point d'émission du faisceau d'illumination. Une telle disposition permet de supprimer sensiblement l'erreur de parallaxe due à la projection du plan du faisceau d'illumination sur le plan de la troisième électrode 14 constituant l'anode.

Dans le mode de réalisation de la figure 1c, les éléments électriquement conducteurs 140 sont formés par des bandes électriquement conductrices formées par des microbandes de cuivre d'une largeur pouvant être comprise entre 80 et 200 µm par exemple et d'une épaisseur de quelques µm. Ces bandes peuvent être réalisées par micro-photogravure et recouvertes d'un film conducteur d'or ou de cuivre. Elles sont réalisées sur un substrat isolant tel qu'un matériau en verre époxy ou une feuille de matière plastique désignée sous la marque commerciale KAPTON par exemple.

En outre, chaque bande 140 est connectée au circuit de détection et de comptage 3 ainsi qu'il sera décrit de façon plus détaillée ultérieurement dans la description. Pour un angle d'ouverture Δθ du faisceau d'illumination dans le plan de l'électrode d'anode 14, chaque électrode élémentaire 140 est alors espacée d'une électrode élémentaire adjacente d'un angle Δθ / N-1 lorsqu'un nombre N d'électrodes est prévu, les électrodes élémentaires 140 étant ainsi espacées de façon régulière dans l'angle d'ouverture du faisceau d'illumination.

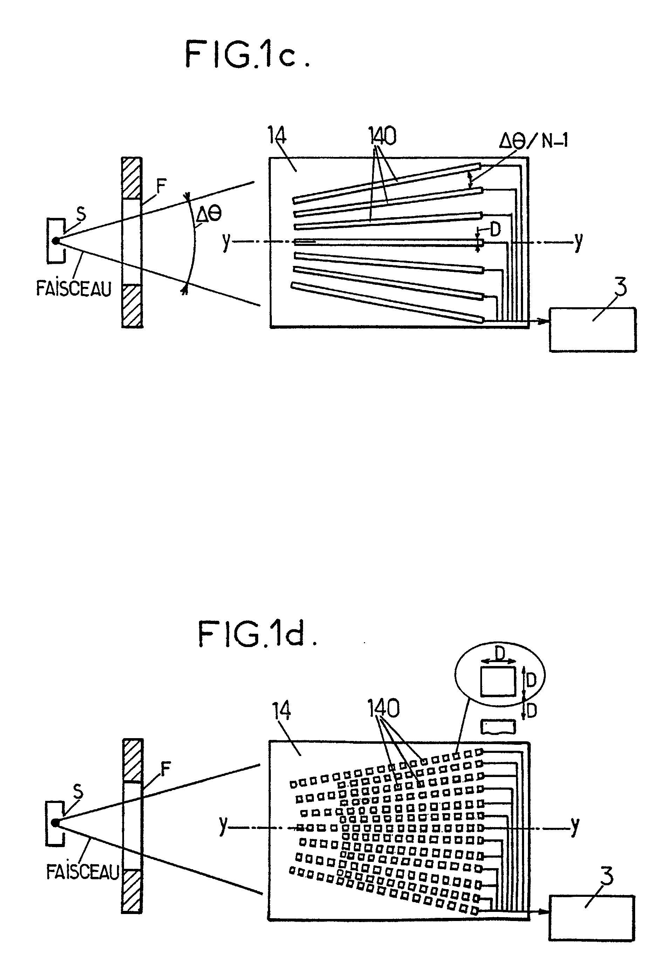

Selon une variante de réalisation représentée en figure 1d, on indique en outre que les éléments électriquement conducteurs constituant les anodes élémentaires 140 peuvent également être constitués de pastilles réparties selon une pluralité d'axes longitudinaux YY, de même que dans le cas de la figure 1c, ces pastilles d'éléments électriquement conducteurs constituant chacune une anode élémentaire et présentant une dimension dans une direction orthogonale à la direction d'axe de symétrie longitudinale YY sensiblement égale à la distance d'amplification. Ainsi, chaque anode élémentaire 140 peut présenter une forme sensiblement carrée dont le côté est égal ou inférieur à la distance de multiplication D, deux anodes élémentaires successives étant espacées par exemple, tant dans la direction longitudinale YY que dans une direction orthogonale à cette direction longitudinale, d'une même distance D. Dans le cas de la figure 1d, on indique que les éléments électriquement conducteurs formant les anodes élémentaires 140 sont également espacés de façon régulière dans l'angle d'ouverture du faisceau d'illumination. Bien entendu, chaque anode élémentaire 140 est elle-même interconnectée au circuit de détection et de comptage 3.

L'avantage de la configuration des anodes élémentaires précitées consiste notamment dans le fait que les phénomènes d'avalanche se développant uniquement sur la distance de multiplication D, il est aisé de choisir la répartition de ces anodes élémentaires pour obtenir la précision la meilleure, alors que dans les chambres à fils une distance entre fils au minimum égale à 1 mm est nécessaire pour assurer un fonctionnement satisfaisant.

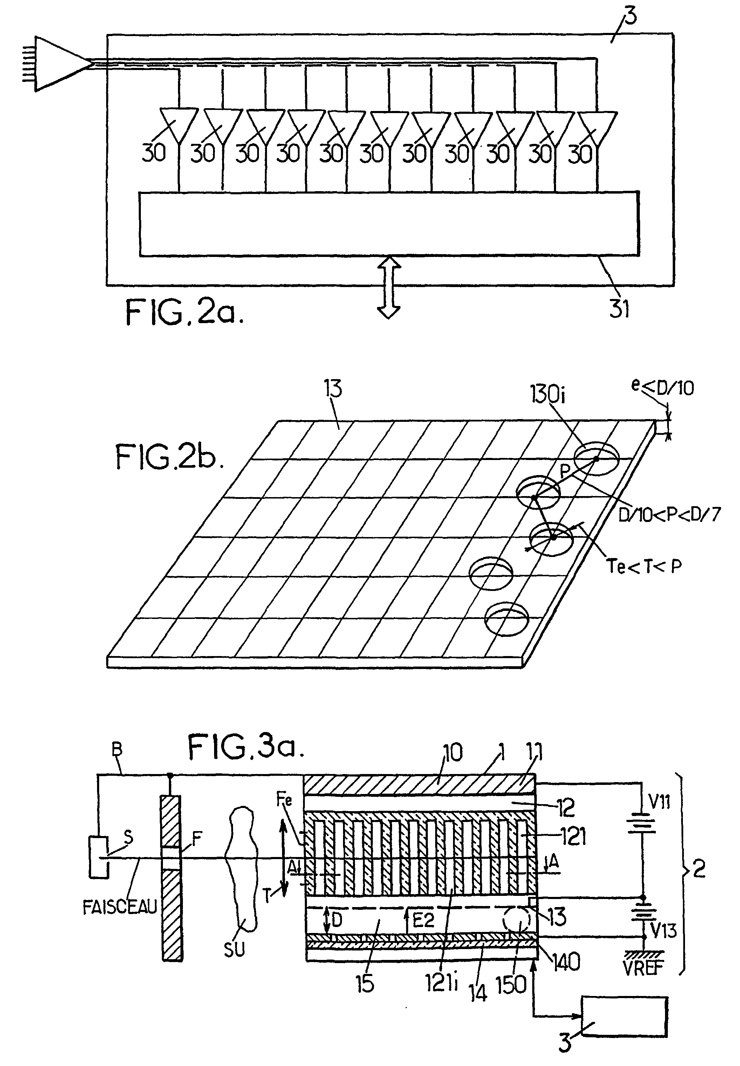

Ainsi que représenté en outre sur la figure 2a, on indique que le circuit 3 de détection et de comptage peut comporter avantageusement une pluralité d'amplificateurs rapides 30, chaque amplificateur rapide 30 étant bien entendu interconnecté à une anode élémentaire 140 et délivrant un signal électrique de détection pour l'anode élémentaire considérée. Enfin, la sortie de chaque amplificateur rapide est interconnectée à un circuit de comptage et de mémorisation 31 par exemple.

Afin d'assurer la transmission des électrons engendrés dans l'espace de conversion, la deuxième électrode 13 formant électrode de cathode est avantageusement formée par une plaque mince électriquement conductrice. Cette plaque est percée de trous représentés de manière non limitative par des trous circulaires sur la figure 2b, chaque trou 130ᵢ étant distant d'un autre trou d'un pas P et présentant une dimension T pour former en fait un réseau de trous tel que représenté sur la figure précitée. L'épaisseur e de la plaque est petite devant la dimension T des trous. Le champ électrique de l'espace de multiplication est alors choisi suffisant pour que les électrons passent à travers la grille.

De manière avantageuse, le pas P du réseau est tel que D/10 < P < D/3.

En outre, la plus grande dimension T de chaque trou 130ᵢ, cette dimension correspondant au diamètre des trous lorsque ceux-ci sont circulaires, est très supérieure à l'épaisseur e, dans un rapport supérieur à 5, et telle que : e « T ≤ P. La grille constitutive de la deuxième électrode 13 formant cathode peut être réalisée par une grille électroformée dont l'épaisseur e peut être de l'ordre de 2 à 5 µm, le diamètre des trous étant par exemple égal à 20 µm et le pas à 25 µm.

Dans un mode d'exécution particulier, la plaque formant la deuxième électrode 13 était formée par une grille d'épaisseur 3 µm, percée de trous carrés de 20 µm, T = 20 µm, espacés au pas P = 25 µm.

Du point de vue du montage mécanique de la deuxième électrode formant cathode 13 et afin d'assurer la tenue mécanique de celle-ci, notamment en présence des forces électrostatiques exercées par les champs de polarisation électrique, des espaceurs électriquement isolants, référencés 150 sur la figure la, doivent pour de grandes surfaces être prévus dans l'espace d'amplification 15 afin de permettre le maintien de la cathode 13 et de l'anode 14. Les espaceurs électriquement isolants peuvent être réalisés par des fils de quartz par exemple dont le diamètre correspond à la distance de multiplication D.

Le dispositif d'imagerie radiographique à haute résolution, objet de l'invention, tel que décrit précédemment dans la description, permet de conduire des analyses de grande précision telles que celles requises pour des applications diagnostiques en mammographie.

Une description plus détaillée d'une variante de réalisation du dispositif d'imagerie radiographique haute résolution, objet de la présente invention, sera maintenant donnée en liaison avec la figure 3a.

Sur la figure précitée, on indique que le dispositif objet de la présente invention comprend, dans l'espace de conversion 12, une structure multicanaux, notée 121, laquelle permet la capture du faisceau d'illumination et la création d'électrons. On indique en particulier que la structure multicanaux 121 est munie d'une pluralité de canaux s'étendant dans la direction du premier champ électrique E1. Le rayonnement ionisant absorbé par la structure multicanaux provoque ainsi la création d'électrons, qui ionisent le gaz qui est dans les canaux et libèrent ainsi des électrons qui sont accélérés dans les canaux correspondants et transmis ensuite par la deuxième électrode de cathode 13 dans l'espace d'amplification 15.

Une particule neutre telle qu'un rayon gamma, pouvant présenter une probabilité très faible d'être absorbé dans l'espace de conversion 12 en l'absence de structure multicanaux, la présence d'une telle structure dans cet espace de conversion 12 permet d'absorber par effet Compton au photoélectrique ou par création de paires un nombre de rayons gamma beaucoup plus important. Les électrons éjectés des parois des canaux ont un parcours limité par l'épaisseur de ces canaux. Ils ionisent le gaz qui est dans le tube et libèrent des électrons qui pénètrent dans l'espace d'amplification 15 ainsi qu'il sera décrit ci-après.

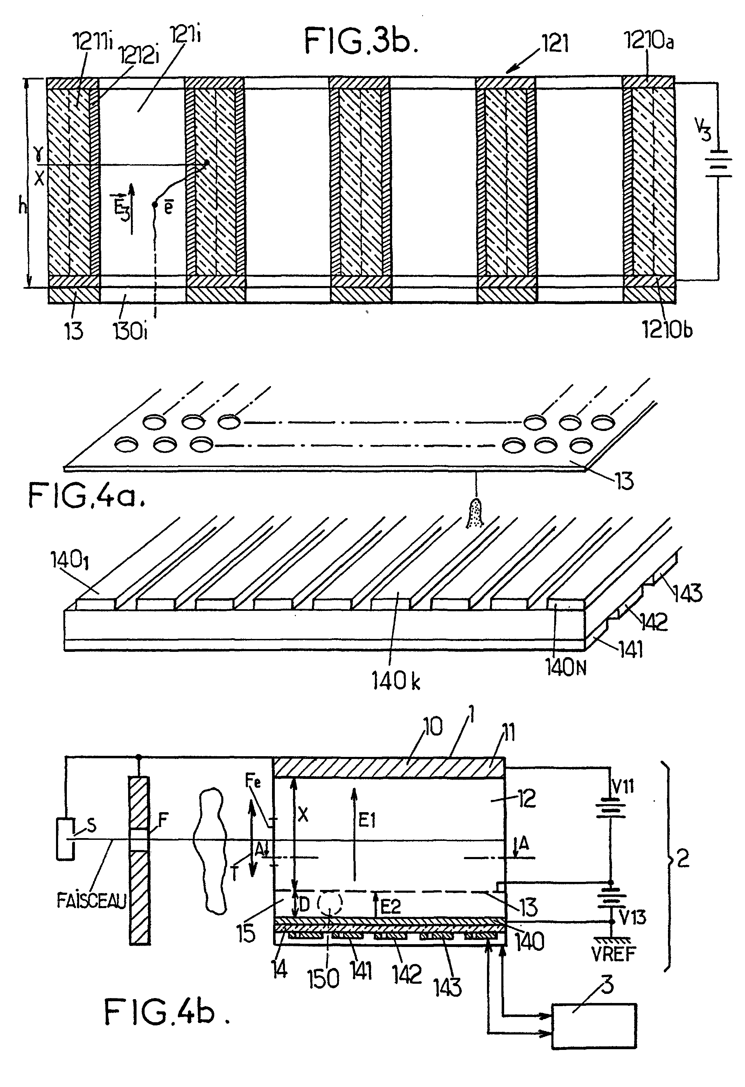

Dans un mode de réalisation préférentiel, ainsi que représenté en figure 3b, la structure 121 est une structure microcanaux munie de canaux parallèles, référencés 121ᵢ, présentant au moins une ouverture abouchant sur une surface plane de cette structure.

Ainsi que représenté en outre sur la figure 3b précitée, l'ensemble de ces ouvertures forme sur la surface plane de la structure microcanaux un réseau de trous au même pas P et à la même dimension T que le réseau de trous 130ᵢ existant sur la grille formant la deuxième électrode 13. Dans un mode de réalisation préférentiel, la face plane de la structure microcanaux présentant l'ensemble des ouvertures de chaque canal est placée au voisinage de la deuxième électrode 13 formant cathode, et, bien entendu, en contact électrique avec celle-ci. Ainsi, une ouverture du réseau d'ouvertures de chaque canal 121ᵢ est placée en vis-à-vis d'un trou de la grille 130ᵢ formant la deuxième électrode 13.

De manière plus spécifique, on indique que la structure 121 représentée en figure 3b peut être formée par des tubes de verre placés côte à côte. Ces tubes de verre peuvent avoir une épaisseur de l'ordre de 2 µm et la paroi de ces derniers porte la référence 1211ᵢ. La hauteur h de l'ensemble, soit la hauteur des tubes de verre, est de l'ordre de 1 à 2 mm.

Un dépôt métallique d'or peut être réalisé sur les faces de la structure microcanaux, dépôt référencé 1210a sur la figure 3b. Le diamètre de chaque canal 121ᵢ, compte tenu du dépôt d'or, peut être compris entre 10 et 30 µm.

La structure microcanaux ainsi réalisée permet, pour des trous de diamètre 12 µm et des parois de tubes de verre de 2 µm par exemple, de constituer une structure dont 25 % du volume est occupé par le verre. Une telle structure présente une densité moyenne de 0,7 g/cm³ alors que, en l'absence de structure microcanaux, le gaz présent dans la chambre, tel que du xénon par exemple, sous pression de 5 bars, présente une densité de 0,03 g/cm³.

L'introduction de la structure microcanaux 121 permet ainsi un gain d'un facteur 20 de la densité moyenne du matériau destiné à capter le rayonnement ionisant, et donc une probabilité de capture augmentée en conséquence.

Avec des tubes d'une paroi d'épaisseur 2 µm, les photoélectrons ou électrons Compton engendrés du fait de la capture ont une importante probabilité de sortir des parois de verre et d'émerger dans les trous ou canaux 121ᵢ, pourvu que ces électrons ou photoélectrons présentent une énergie supérieure à quelques keV.

Conformément à un aspect particulièrement avantageux du dispositif de structure microcanaux représenté en figure 3b, un champ électrique E₃ peut alors être créé au sein de chaque trou, ce champ électrique étant sensiblement longitudinal et parallèle à l'axe de chaque trou 121ᵢ. Dans ce but, les parois de chaque canal 121ᵢ peuvent comporter un revêtement 1212ᵢ électriquement conducteur et présentant une résistivité élevée. Ce revêtement est électriquement connecté à des électrodes constituées par les revêtement d'or 1210ₐ et 1210_{b} placées chacune sur la face plane de la structure microcanaux et une tension de dérive des électrons dans les canaux 121ᵢ est appliquée aux électrodes 1210a et 1210b. Chaque électron émergeant dans l'un des canaux 121ᵢ est alors soumis à un phénomène de dérive du fait de l'existence du champ électrique E₃ représenté sur la figure 3b. Les revêtements 1212ᵢ de chaque canal 121ᵢ peuvent être formés par un film de plomb de quelques nanomètres d'épaisseur. Le champ électrique E3 peut être créé par une source de tension appropriée V₃. Les électrons d'ionisation libérés dans les canaux de la structure microcanaux peuvent en outre être multipliés, préalablement à leur transfert dans l'espace d'amplification 15, en utilisant l'espace de conversion comme une des chambres multi-étages telles que proposées par Monsieur Georges CHARPAK. D'une manière plus spécifique, on indique que la structure microcanaux précitée peut être utilisée soit en admission latérale du faisceau d'illumination, ainsi que représenté en figures 3a et 4b, soit en admission frontale du faisceau d'illumination, ainsi que représenté en figure 1b, afin de détecter des rayons X mous, en particulier en vue de conduire des travaux d'analyse en cristallographie.

Une description plus détaillée d'un mode de réalisation particulier du circuit de détection et de comptage des électrons multipliés permettant une détection bidimensionnelle, réalisée en particulier par la mise en oeuvre d'un circuit à couplage par influence électrique avec les anodes élémentaires précédemment décrites dans la description, sera maintenant donnée en liaison avec les figures 4a à 4d.

Ainsi que représenté sur la figure 4a, les anodes élémentaires peuvent être constituées par des premières bandes conductrices, parallèles, 140ₗ, 140ₖ à 140_{N}, portées au même potentiel électrique V_{REF}, ainsi que décrit précédemment dans la description.
En outre, un circuit à couplage par influence électrique est formé par des deuxièmes bandes conductrices 141, 142 ...143 et suivantes, ces deuxièmes bandes étant orthogonales aux premières, espacées régulièrement, et donnant lieu, par un phénomène d'influence électrique de charges en mouvement à un signal de signe opposé à celui apparaissant sur les premières bandes 140_{k.}
Compte tenu du couplage par influence électrique précité, les électrons collectés sur une bande conductrice 140ₖ par exemple rayonnent sur une ou plusieurs bandes 141, 142, 143 ..., celles situées proches de l'impact des électrons sur la bande 140ₖ, la bande 142 par exemple, un champ électrostatique variable qui disparaît soudainement lorsque ces électrons sont collectés par la bande conductrice 140ₖ, ce qui permet de produire sur les bandes 140ₖ et 142 une impulsion de position de signe positif et un repérage bidimensionnel X,Y du point d'impact. Les ions positifs qui sont produits sur les bandes 140ₖ, anodes élémentaires, et s'éloignent de celles-ci, induisent des signaux négatifs sur ces dernières mais les lignes de champ électrostatique rayonnées sur les deuxièmes bandes 141 à 143 et suivantes sont d'abord positives, ce qui permet de distinguer les types d'impulsions X,Y et donc la détection bidimensionnelle.
A titre d'exemple non limitatif, les première et deuxième bandes conductrices peuvent être formées sur un substrat de matériau isolant d'épaisseur appropriée, le dimensionnement en épaisseur, largeur et espacement des bandes étant déterminé expérimentalement.

Ainsi qu'on l'observera sur la figure 4b, on constate que la troisième électrode d'anode 14 peut être formée sur une plaquette de matériau électriquement isolant, genre circuit imprimé, sur la face supérieure de laquelle, en vis-à-vis de l'espace d'amplification 15, sont placées les électrodes élémentaires 140ₖ précédemment décrites dans la description.

Dans le mode de réalisation précité, on indique que le circuit de détection et de comptage 3 peut comporter en outre un circuit de détection électrostatique, placé dans le prolongement des bandes 140ₖ constitutives des anodes élémentaires, variante de réalisation de celui représenté sur la figure 4a, chaque référence 1410, 1420, 1430 correspondant par exemple à une bande électriquement conductrice. Cette bande électriquement conductrice est subdivisée selon un motif périodique en deux bandes élémentaires dont le couplage électrostatique à chaque anode élémentaire 140ₖ est une fonction croissante, respectivement décroissante, du rang k de l'anode élémentaire à laquelle chaque bande élémentaire 1410, 1420, 1430 est couplée.

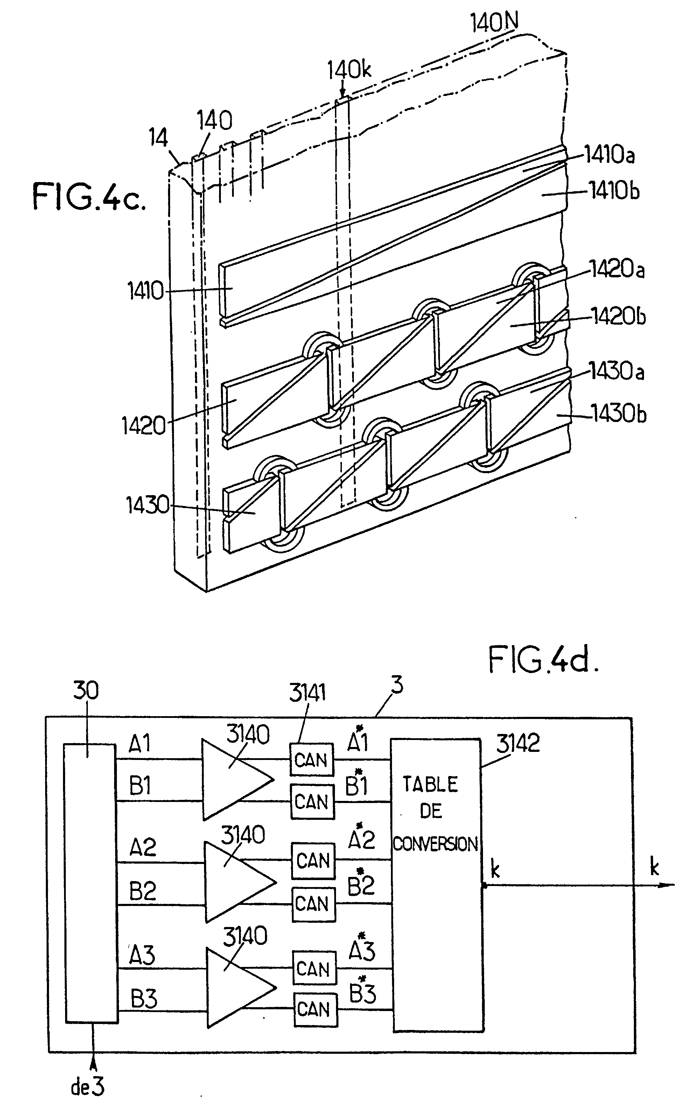

Ainsi qu'on l'a représenté de manière plus détaillée en figure 4c, la bande élémentaire 1410 par exemple est subdivisée en deux bandes élémentaires 1410ₐ, 1410_{b} dont le couplage électrostatique à chaque anode élémentaire 140ₖ est une fonction croissante, respectivement décroissante, du rang de la bande considérée.

Un circuit, tel que représenté en figure 4d, permet de mesurer l'amplitude du signal d'extrémité engendré en extrémité de la première 1410ₐ et de la deuxième 1410_{b} bande élémentaire, ainsi que du rapport des signaux d'extrémité. Ce rapport est représentatif du rang de l'anode élémentaire, rang k, au voisinage de laquelle les électrons d'ionisation ont induit l'impulsion électrique correspondante.

Ainsi que représenté sur la figure 4c, on indique qu'une deuxième bande 1420 peut être prévue, cette bande étant également une bande électriquement conductrice et subdivisée en une première, 1420ₐ, et deuxième, 1420_{b}, bande élémentaire selon un même motif périodique de périodes multiples de la première bande électriquement conductrice 1410 dans un rapport donné.

En outre, une troisième bande 1430 peut être prévue, elle-même subdivisée en bandes élémentaires 1430ₐ, 1430_{b}. La période spatiale de la troisième bande 1430 est également un multiple de la première bande électriquement conductrice dans un autre rapport.

Le circuit représenté en figure 4d permet d'effectuer la mesure de l'amplitude des signaux engendrés par le couplage capacitif en extrémité de la première, 1410, et de la deuxième, 1420, bande élémentaire et du rapport de l'amplitude de ces signaux d'extrémité. La première et la deuxième bande électriquement conductrices 1410, 1420, associées le cas échéant à la troisième bande électriquement conductrice 1430, elle-même subdivisée en deux bandes élémentaires 1430ₐ, 1430_{b}, forment ainsi un système de mesurage, de type vernier, du rang k de chaque anode élémentaire 140ₖ située sensiblement au droit des électrons d'ionisation premièrement émis. En figure 4c, le circuit 3 de détection de comptage est réputé comprendre un circuit logique de type interrupteur commandé, les circuits 3140 désignent des circuits amplificateurs, les circuits 3141 des convertisseurs analogiques numériques et le circuit 3142 une table de conversion permettant de délivrer le rang k de chaque anode élémentaire 140ₖ sur laquelle l'impulsion a été détectée.

Pour une description plus détaillée du fonctionnement du système de détection électrostatique représenté en figures 4c et 4d, on pourra se reporter à la demande de brevet français FR-A 2 680 010 au nom de Monsieur Georges CHARPAK.

Enfin on indique qu'un jeu d'électrodes 1410 à 1430 formant un circuit de détection capacitif, pour une détection monodimensionnelle des anodes élémentaires 140ₖ, peut être prévu pour réaliser de la même manière un circuit de détection capacitif pour une détection monodimensionnelle des deuxièmes bandes élémentaires 140, 141, 142... et suivantes, dans la deuxième direction. Ce jeu d'électrodes est alors placé sur un prolongement des bandes élémentaires 140, 141, 142 et suivantes, de même que dans le cas des anodes élémentaires 140ₖ.

Pour la mise en oeuvre des dispositifs tels que représentés en figures 1a, 1b à 4d précédemment décrits dans la description, on indique que le gaz contenu dans l'enceinte à gaz peut être le xénon mélangé à un agent de "quenching" tel que CO₂ ou l'éthane à une pression comprise entre 10² et 10⁶ Pa ou, le cas échéant, un mélange d'argon et de méthane à raison de 90% d'argon et 10% de méthane en volume.

Toutefois, le gaz contenu dans l'enceinte à gaz 10 peut être constitué par un mélange d'argon et de triethylamine, ceci afin de permettre d'engendrer dans l'espace d'amplification 15, une multiplication des électrons par phénomène d'avalanches lumineuses.

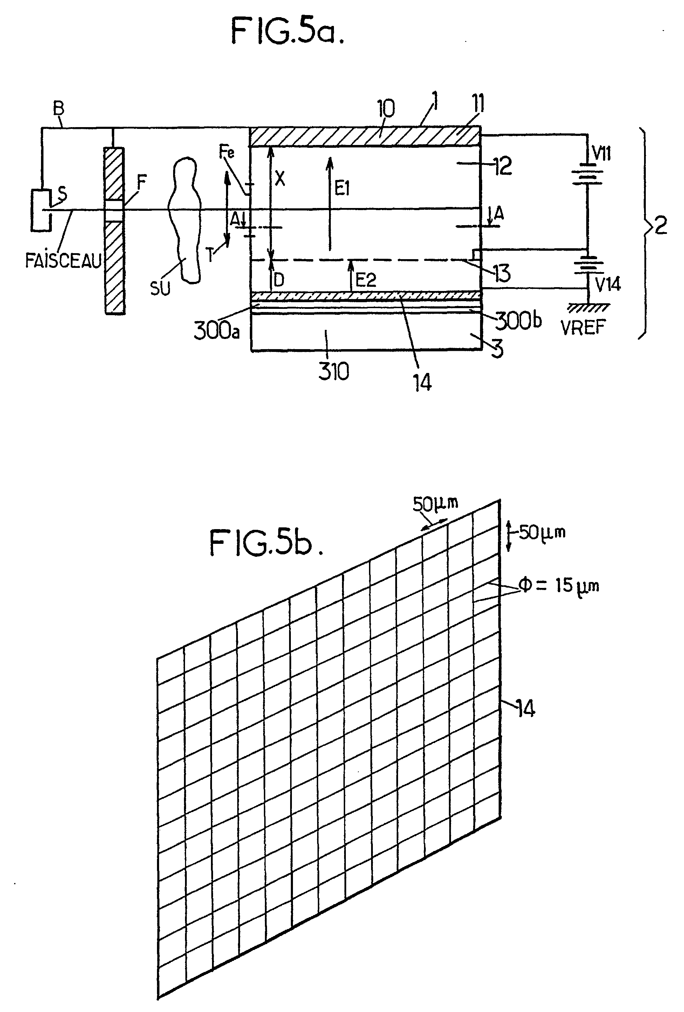

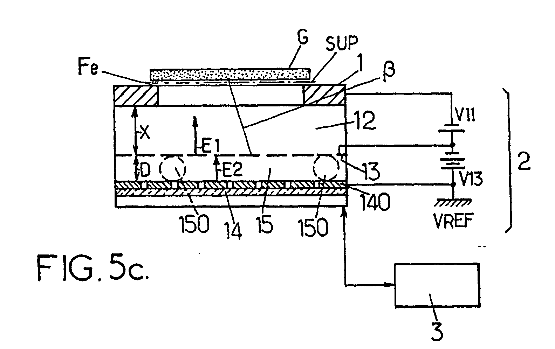

Dans un tel cas, ainsi que représenté en figure 5a, les anodes élémentaires sont formées par des éléments électriquement conducteurs transparents permettant la transmission de la lumière engendrée par les avalanches lumineuses ainsi créées.

Dans le mode de réalisation décrit en liaison avec la figure 5a, les circuits de détection et de comptage 3 sont par exemple constitués par un amplificateur de brillance 300_{b}, équipé d'une optique appropriée 300ₐ, suivi d'un système intégrateur 310, tel qu'un film photosensible ou un système de caméra CCD par exemple. L'ensemble 300ₐ, 300_{b}, 310 amplificateur de brillance caméra CCD est alors placé sur la paroi de l'électrode permettant la transmission ou le changement de la longueur d'onde du phénomène d'avalanches lumineuses ainsi engendrées, 280 nm dans le cas du triethylamine en lumière visible.

A titre d'exemple non limitatif, ainsi que représenté en figure 5b, on indique que les anodes élémentaires peuvent être formées par une grille électriquement conductrice associée à une paroi permettant, soit la transmission de la lumière à la longueur d'onde du phénomène d'avalanche lumineuse, soit le changement de la longueur d'onde du phénomène d'avalanche lumineuse en une longueur d'onde du spectre du domaine visible. A titre d'exemple non limitatif, la grille électriquement conductrice telle que représentée en figure 5b peut être formée par des fils conducteurs, des fils de tungstène doré de diamètre 15 µm au pas rectangulaire de 50 µm x 50 µm. Chaque zone élémentaire transparente de 50 µm constitue en fait l'équivalent d'une anode élémentaire au travers de laquelle l'énergie lumineuse de l'avalanche lumineuse créée est transmise. Cette grille peut également être constituée par une grille semblable à la deuxième électrode 13 représentée en figure 2b. La grille précitée est alors placée contre un convertisseur de lumière UV visible. Bien entendu, la grille telle que représentée en figure 5b est portée au potentiel de référence V_{REF} et constitue ainsi l'anode constituée d'un ensemble d'anodes élémentaires transparentes. L'enceinte à gaz est alors fermée par l'intermédiaire d'une fenêtre de quartz ou d'une fenêtre en matériau plastique par exemple.

L'avantage du mode de réalisation de la figure 5a dans lequel une détection des phénomènes d'avalanches lumineuses est réalisée, vis-à-vis des dispositifs de l'art antérieur assurant de même une détection des avalanches lumineuses, réside dans le fait que les fronts d'avalanches étant beaucoup plus étroits, une plus grande précision est obtenue relativement à leur position ainsi qu'une meilleure brillance.
Cette amélioration apparaît d'une très grande importance lorsque la détection est réalisée non au coup par coup, au moyen de circuits électroniques, mais grâce à une intégration des photons lumineux. Un tel mode opératoire est en particulier important lorsque la source d'émission S délivre une salve très intense de rayons X ou γ, source d'émission telle qu'un accélérateur linéaire utilisé en radiothérapie. Dans un tel cas, il peut être nécessaire de visualiser simultanément des centaines de milliers ou de millions d'avalanches, ce qui n'est pas réalisable avec des moyens électroniques classiques. Il est alors possible d'utiliser comme convertisseur particules ionisantes-électrons une structure microcanaux, telle que décrite précédemment dans la description, ou une matrice au plomb-bismuth telle que décrite dans l'article publié par A.P.JEAVONS, G.CHARPAK et R.J.STUBBS, Nuclear Instruments and Methods 124 (1975), 491-503, North-Holland Publishing and Co. Une efficacité de 10 à 50% pour des rayons gamma de 6 MeV couramment utilisés en radiothérapie peut être obtenue avec une précision d'image de 0,5 mm à 1 mm. Il est ainsi possible de réaliser une radiographie du patient, lors du traitement de ce dernier, en le soumettant à une dose de rayonnement très inférieure à celle du traitement. Ainsi, la détection lumineuse constitue un moyen d'obtenir une grande précision, en particulier lorsqu'une optique appropriée 300ₐ, un amplificateur de brillance 300_{b} et une caméra CCD par exemple, 310, sont mis en oeuvre.
Dans ce mode d'utilisation, le faisceau d'illumination peut être admis soit latéralement, conforméméent au mode de représentation de la figure 1a, soit frontalement, ainsi que représenté en figure 1b.
Dans le cas d'une admission latérale du faisceau d'illumination, une mesure peut être effectuée toutes les 50 ms, soit une durée de trame de la caméra CDD utilisée, alors qu'un déplacement du module de détection est réalisé.
Toutefois, une admission frontale apparaît particulièrement appropriée lorsque le module de détection comprend, dans l'espace de conversion 12, un convertisseur microcanaux et que l'on procède à la saisie de l'image de toutes les avalanches engendrées par une impulsion ou *"flash"* de rayons γ, tel qu'utilisé en radiothérapie au moyen d'un accélérateur linéaire, émettant des rayons γ pendant des temps très courts. Dans le cadre d'une telle application, les avalanches lumineuses étant très étroites, on obtient directement une bonne résolution d'image en l'absence de toute nécessité de procéder à un traitement d'image tel que le calcul du centroïde, centre de gravité optique, de chaque avalanche, ainsi que proposé antérieurement par Monsieur Georges CHARPAK. Ce mode opératoire permet donc d'obtenir une économie importante en matériel et temps de calcul, vis-à-vis des dispositifs antérieurs.

Outre les applications précitées en radiographie X ou gamma, le dispositif objet de la présente invention peut être utilisé avec un intérêt majeur en radiographie β, dans des conditions spécifiques qui seront décrites ci-après, en liaison avec la figure 5c. Ainsi que représenté sur la figure précitée, dans cette application, la source de rayonnement ionisant consiste alors en un gel d'électrophorèse, une coupe ou une préparation, portant la référence G, le gel, la coupe ou la préparation étant marqué radioactivement de manière classique. Le gel d'électrophorèse G est alors le siège de points d'émission de rayons β distribués sur la section du gel, de la coupe, ou de la préparation, le faisceau d'illumination consistant alors en une pluralité de rayons β émis sporadiquement en ces différents points d'émission. Le gel G est placé au voisinage de la fenêtre Fe pour une admission frontale du faisceau d'illumination. Un support SUP, représenté en traits mixtes sur la figure 5c permet, si nécessaire, de placer le gel, la coupe ou la préparation pour observation.

Selon une caractéristique, spécifique du détecteur objet de la présente invention, celui-ci est tel que la première électrode 11 et la deuxième électrode 13 sont distantes d'une distance X voisine ou inférieure à celle de la distance D séparant la deuxième électrode 13 de la troisième électrode 14. L'espace de conversion 12 et l'espace d'amplification 15 présentent ainsi une dimension sensiblement égale dans la direction perpendiculaire aux électrodes 11, 13 et 14.

A titre d'exemple non limitatif, pour une distance D = 100 µm la distance X peut être prise égale à cette valeur X = 100 µm. L'épaisseur totale du détecteur 1 est alors de 0,2 mm.

Dans ces conditions, un rayon β émis à partir d'un point d'émission quelconque du gel G présente, ainsi que représenté en figure 5c, un parcours maximum de l'ordre de grandeur de la distance X séparant la première et la deuxième électrode 11, 13. Les électrons étant engendrés dans l'espace de conversion 12, sur le trajet du rayon β, la précision de localisation du point d'émission correspondant est meilleure que la valeur de cette distance X. Le dispositif détecteur objet de la présente invention permet de développer des appareils plus simples que les appareils actuels à chambres à faces parallèles dans lesquels la localisation du point d'émission du rayon β est effectuée grâce au gain variable en fonction de la profondeur de l'électron d'ionisation le long de la coordonnée z.

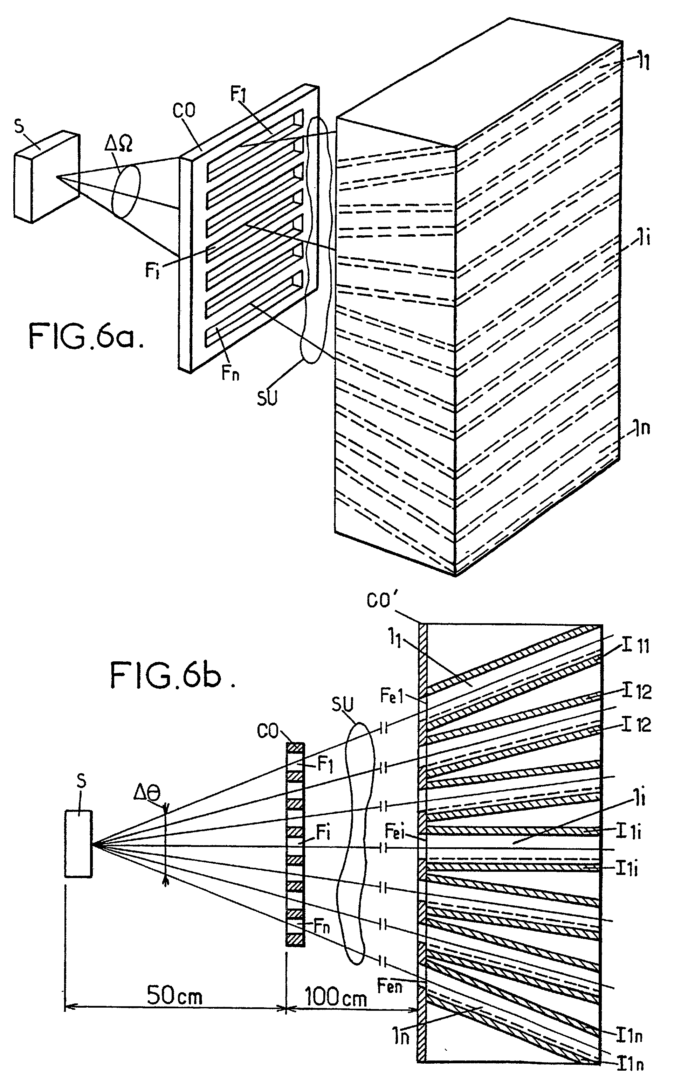

Une description plus détaillée d'un dispositif d'imagerie radiographique en rayonnement ionisant X ou γ à haute résolution, permettant la réalisation d'analyse d'images de type tridimensionnel en une seule exposition, sera maintenant donnée en liaison avec les figures 6a et 6b.

Selon la figure 6a précitée, la source de rayonnement utilisée S est réputée engendrer un faisceau de rayonnement ionisant selon un faisceau divergent d'angle solide donné ΔΩ.

Selon le mode de réalisation de la figure 6a, le dispositif d'imagerie radiographique selon l'invention comprend un collimateur CO comportant une pluralité de fentes longitudinales, notées Fₗ, ... Fᵢ, ... Fₙ, formant une pluralité de diaphragmes permettant de délivrer en fait une pluralité de faisceaux d'illumination en nappe ainsi que représenté sur la figure précitée. Chaque faisceau d'illumination en nappe est distribué sensiblement dans un plan contenant l'une des fentes longitudinales de l'ensemble de ces fentes et la source S. Ainsi que représenté sur la figure 6a, l'ensemble des plans contenant l'un des faisceaux d'illumination en nappe forme un faisceau de plans convergeant vers la source de rayonnement ionisant.

Ainsi que représenté également sur la figure précitée, le dispositif selon l'invention comprend un module de détection de l'ensemble des faisceaux d'illumination en nappe, lesquels ont permis d'illuminer le sujet à observer SU, ce sujet étant placé en aval du collimateur CO. Ce module de détection comprend un détecteur de particules ionisantes tel que précédemment décrit dans la description en liaison avec les figures 1a à 5b, ce détecteur 1 étant placé en vis-à-vis d'un faisceau d'illumination en nappe correspondant. Ainsi, chaque détecteur, noté lₗ, lᵢ à lₙ, correspond à une fente Fₗ, Fᵢ à F_{n,} et en particulier, la fenêtre Feₗ, Feᵢ à Feₙ de chacun de ces détecteurs, fenêtre d'admission du faisceau d'illumination en nappe, est placée dans le plan formé par la source de rayonnement ionisant S et la fente Fi délivrant ce faisceau d'illumination en nappe auquel ce détecteur est associé. Les fenêtres Feᵢ peuvent être creusées, ainsi que représenté en figure 6b, dans un collimateur commun CO'.

Ainsi qu'on l'a représenté sur les figures 6a et 6b, on peut constater que l'ensemble des détecteurs lₗ à lₙ est incliné symétriquement par rapport à un détecteur central, le détecteur lᵢ sur les figures précitées.

Afin d'assurer l'indépendance de chaque détecteur lᵢ, ceux-ci peuvent être munis de protections, notées Iₗᵢ, protections constituées par une feuille de plomb ou tungstène permettant d'assurer l'indépendance de chacun des détecteurs lᵢ vis-à-vis des rayonnements ionisants diffusés par chaque faisceau d'illumination en nappe voisin.

On a ainsi décrit un dispositif d'imagerie radiographique à haute résolution particulièrement performant dans la mesure où le détecteur 1 mis en oeuvre présente une très faible épaisseur. En effet, compte tenu de l'espace de de conversion 12 et de l'espace d'amplification 15, l'épaisseur totale est de l'ordre de 1,1 mm. En particulier, cette caractéristique spécifique du dispositif d'imagerie radiographique à haute résolution, objet de la présente invention, permet la mise en oeuvre d'un appareil comportant des détecteurs multiples tels que représentés en figures 6a et 6b. Ces détecteurs fonctionnent en quelque sorte en parallèle et la multiplication des détecteurs, lorsqu'un nombre n de détecteurs est utilisé, permet de gagner dans le temps de balayage du sujet SU à observer, dans un rapport n correspondant.

On comprend bien sûr que l'ensemble des n détecteurs peut être placé dans une enceinte à gaz unique sous pression avec des écrans de séparation en plomb, les écrans Iₗᵢ précédemment mentionnés dans la description, ces derniers empêchant tout rayonnement secondaire produit dans un détecteur d'aller perturber la détection dans les détecteurs voisins.

Enfin, dans le cas où le rayonnement ionisant est un rayonnement à haute énergie tel qu'un rayonnement γ d'énergie supérieure à 1 MeV, susceptible d'être utilisé en radiographie industrielle, les détecteurs 1 mis en oeuvre peuvent comporter, en lieu et place de la structure microcanaux 12, précédemment mentionnée dans la description, une pluralité de feuilles minces inclinées, telles que des feuilles de tantale placées sous incidence rasante, de l'ordre de 1 degré, par rapport à la direction moyenne du flux d'illumination de particules ionisantes, selon une technique connue de l'art antérieur. Ces feuilles permettent d'assurer la conversion des particules.
Ce mode de mise en oeuvre permet, d'une part, d'augmenter la probabilité de capture des particules ionisantes et d'éjection des photo-électrons ainsi engendrés, conformément à la technique de l'art antérieur précitée, mais, d'autre part, grâce à la très grande capacité de taux de comptage des détecteurs 1 conformes à l'objet de la présente invention utilisés, de simplifier et de réduire considérablement les coûts de mise en oeuvre de l'accélérateur de particules générateur des particules ionisantes utilisées.

## Revendications

1. Dispositif d'imagerie radiographique en rayonnement ionisant à haute résolution, comportant une source de rayonnement ionisant (S) selon un faisceau divergent, une fente longitudinale (F) formant diaphragme permettant de délivrer un faisceau d'illumination en nappe distribué sensiblement dans un plan contenant la fente longitudinale (F) et des moyens de détection d'un faisceau transmis par un sujet à observer (SU), illuminé par le faisceau d'illumination en nappe comprenant une enceinte à gaz (10) munie d'une fenêtre d'admission (Fe) dudit faisceau d'illumination, **caractérisé en ce que** lesdits moyens de détection comportent au moins un détecteur (1) de particules ionisantes comprenant au moins, dans cette enceinte à gaz :
- une première (11), deuxième (13) et troisième (14) électrode plane, chaque électrode plane étant placée parallèlement chacune à chacune pour former d'une part entre les première (11) et deuxième (13) électrodes, un espace de conversion (12) du faisceau d'illumination en électrons et, d'autre part, entre les deuxième (13) et troisième (14) électrodes, un espace d'amplification (15) par multiplication de ces électrons, ladite fenêtre d'admission (Fe) étant placée au niveau de l'espace de conversion pour assurer l'admission dudit faisceau d'illumination dans l'espace de conversion, ledit espace d'amplification (15) présentant une longueur de multiplication, distance D séparant la deuxième (13) et la troisième électrode (14), inférieure à 200 µm, ce détecteur comportant en outre :
- des moyens (2) de polarisation permettant de porter la première électrode (11) à un premier potentiel électrique, la deuxième électrode (13) à un deuxième potentiel électrique, supérieur au premier potentiel électrique, et la troisième électrode (14) à un troisième potentiel électrique supérieur au deuxième potentiel électrique, la deuxième électrode (13) étant percée de trous formant une cathode permettant la transmission des électrons engendrés dans l'espace de conversion (12) vers l'espace d'amplification (15), et la troisième électrode (14) formant une anode constituée par une pluralité d'anodes élémentaires isolées électriquement, les potentiels électriques appliqués à la première (11), à la deuxième (13) et à la troisième électrode (14) permettant d'engendrer un premier champ électrique E₁ dans l'espace de conversion (12) et un deuxième champ électrique E₂ sensiblement parallèle dans l'espace d'amplification (15), ces champs électriques E₁, E₂ étant dans un rapport d'amplitude ρ=E₂/E₁ supérieur à 10, chacun de ces champs électriques E₁ et E₂ étant sensiblement orthogonal auxdites électrodes, ce qui permet d'assurer la multiplication de ces électrons dans l'espace d'amplification par phénomène d'avalanche confiné à la longueur d'amplification D pour engendrer, au voisinage de la troisième électrode (14) formant anode, des électrons multipliés,
- des moyens (3) de détection et de comptage des électrons multipliés couplés auxdites anodes élémentaires formant l'anode (14).

2. Dispositif selon la revendication 1, **caractérisé en ce que** ladite fenêtre d'admission (Fe) est placée, soit latéralement sur l'enceinte à gaz (10) de façon à permettre l'admission dudit faisceau d'illumination parallèlement aux électrodes, soit sur la face de l'enceinte à gaz (10) située en vis-à-vis de la deuxième électrode (13) formant électrode de cathode de façon à permettre une admission frontale dudit faisceau d'illumination vis-à-vis des électrodes.

3. Dispositif selon la revendication 1 ou 2, **caractérisé en ce que** lesdites anodes élémentaires sont formées par des éléments électriquement conducteurs (140) présentant un axe de symétrie longitudinale, l'axe de symétrie longitudinale de chaque élément électriquement conducteur (140) étant orienté de manière à converger vers la source de rayonnement ionisant (S), point d'émission du faisceau d'illumination, ce qui permet de supprimer sensiblement l'erreur de parallaxe due à la projection du plan du faisceau d'illumination sur le plan de la troisième électrode (14) constituant l'anode.

4. Dispositif selon la revendication 2 ou 3, **caractérisé en ce que** lesdits éléments électriquement conducteurs (140) constituant ces anodes élémentaires présentent une dimension dans une direction orthogonale à ladite direction d'axe de symétrie longitudinale sensiblement égale à la distance d'amplification (D), deux éléments électriquement conducteurs consécutifs selon cette même direction orthogonale étant espacés de façon régulière dans l'angle d'ouverture (Δθ) du faisceau d'illumination.

5. Dispositif selon l'une des revendications 1 à 4, **caractérisé en ce que** lesdits moyens (3) de détection et de comptage comportent :
- une pluralité d'amplificateurs rapides (30), un amplificateur rapide étant couplé à un élément électriquement conducteur formant une anode élémentaire (140), et délivrant un signal électrique de détection pour l'anode élémentaire (140) considérée ;
- des moyens (31) de comptage et de mémorisation interconnectés en sortie des amplificateurs rapides.

6. Dispositif selon l'une des revendications 1 à 5, **caractérisé en ce que** ladite deuxième électrode (13) est formée par une grille électriquement conductrice, et **en ce que** la grille présente un réseau régulier de trous (130i) dont le pas P, dans le plan de la deuxième électrode est compris entre D/10 et D/3, la plus grande dimension T des trous étant supérieure à l'épaisseur e de cette grille mais inférieure à P.

7. Dispositif selon l'une des revendications 1 à 6, **caractérisé en ce que** celui-ci comprend en outre, dans l'espace de conversion (12), une structure multicanaux (121) permettant la capture du faisceau d'illumination et la création d'électrons, chaque canal de la structure multicanaux s'étendant dans la direction du premier champ électrique (E₁).

8. Dispositif selon la revendication 7, **caractérisé en ce que** ladite structure multicanaux (121) est une structure microcanaux formée par un bloc en matériau absorbant le rayonnement ionisant du faisceau d'illumination, cette structure étant munie de microcanaux parallèles (121i) présentant au moins une ouverture abouchant sur une surface plane de cette structure, l'ensemble de ces ouvertures formant sur cette surface plane un réseau de trous au même pas P et à la même dimension T que le réseau de trous dans la grille formant la deuxième électrode (13), ladite surface plane et ce réseau d'ouvertures étant placés au voisinage de cette deuxième électrode (13) formant cathode, une ouverture de ce réseau d'ouvertures étant placée en vis-à-vis d'un trou (130i) de la grille formant cette deuxième électrode, les ouvertures et les trous étant ainsi en vis-à-vis.

9. Dispositif selon l'une des revendications précédentes, **caractérisé en ce que** lesdits moyens de détection et de comptage comportent en outre un circuit de détection électrostatique consistant en un couplage capacitif avec chaque anode élémentaire (140ₗ, ... ,140ₖ... 140_{N}), ce circuit de détection électrostatique comportant au moins une première bande (1410 à 1430) électriquement conductrice, cette bande électriquement conductrice étant subdivisée selon un motif périodique en deux bandes élémentaires (1410a,1410b) dont le couplage électrostatique à chaque anode élémentaire (140ₗ,...,140ₖ...140_{N}) est une fonction croissante respectivement décroissante du rang k de l'anode élémentaire à laquelle chaque bande élémentaire est couplée.

10. Dispositif selon l'une des revendications 1 à 9, **caractérisé en ce que** le gaz contenu dans l'enceinte à gaz est le xénon mélangé à une vapeur de "quenching" à une pression comprise entre 10² et 10⁶ Pa.

11. Dispositif selon la revendication 1, **caractérisé en ce que** le gaz contenu dans l'enceinte à gaz est constitué par un mélange d'argon et de triméthylamine permettant d'engendrer dans ledit espace d'amplification une multiplication des électrons par phénomène d'avalanches lumineuses, lesdites anodes élémentaires (140) étant formées par des éléments électriquement conducteurs transparents, permettant la transmission de la lumière engendrée par les avalanches lumineuses ainsi créées.

12. Dispositif selon la revendication 11, **caractérisé en ce que** lesdites anodes élémentaires (140) sont formées par une grille électriquement conductrice associée à une paroi permettant soit la transmission de la lumière à la longueur d'onde du phénomène d'avalanche lumineuse, soit le changement de la longueur d'onde du phénomène d'avalanche lumineuse en une longueur d'onde du spectre du domaine visible.

13. Dispositif selon la revendication 12, **caractérisé en ce que** les moyens de comptage sont constitués par des moyens intégrateurs, tels qu'un film sensible, associés à des moyens optiques (300a) et amplificateur de brillance (300b), placés sur la paroi permettant la transmission ou le changement de la longueur d'onde du phénomène d'avalanche lumineuse.

14. Dispositif selon la revendication 1 ou 2, **caractérisé en ce que** pour une imagerie en radiographie , la source de rayonnement ionisant consistant en un gel d'électrophorèse ou une coupe radioactivement marqué siège de points d'émission de rayons β et placé au voisinage de la fenêtre pour une admission frontale du faisceau d'illumination, lesdites première (11) et deuxième (13) électrode sont distantes d'une distance X égale ou inférieure à celle de la distance séparant la deuxième (13) et la troisième électrode (14), l'espace de conversion et l'espace d'amplification présentant une dimension sensiblement égale dans une direction perpendiculaire auxdites électrodes (11, 13, 14), ce qui permet de limiter la longueur du parcours de chaque rayon β émis par la source à une valeur sensiblement égale à celle de la distance X séparant la première (11) et la deuxième électrode (13) et d'amener la précision de localisation des points d'émission de rayons β à une valeur sensiblement égale ou inférieure à cette distance X.

15. Dispositif d'imagerie radiographique en rayonnement ionisant à haute résolution, comportant une source de rayonnement ionisant selon un faisceau divergent, **caractérisé en ce qu'**il comprend :
- un collimateur (CO) comportant une pluralité de fentes longitudinales (F₁ à Fₙ) formant une pluralité de diaphragmes permettant de délivrer une pluralité de faisceaux d'illumination en nappe, chacun distribué sensiblement dans un plan contenant une des fentes longitudinales de cette pluralité de fentes longitudinales, l'ensemble des plans contenant l'un des faisceaux d'illumination en nappe formant un faisceau de plans convergents vers ladite source de rayonnement ionisant (S), et
- des moyens de détection de l'ensemble des faisceaux d'illumination en nappe, lesdits moyens de détection comprenant un détecteur (1₁ à 1ₙ) de particules ionisantes selon l'une des revendications 1 à 13, placé en vis-à-vis de chaque faisceau d'illumination en nappe, chaque détecteur étant placé dans le plan formé par la source de rayonnement ionisant et la fente délivrant en nappe auquel ce détecteur est associé.

## Patentansprüche

1. Hochauflösende Vorrichtung zur radiographischen Bilderzeugung mit ionisierender Strahlung, umfassend eine ionisierende Strahlungsquelle (S) mit einem divergierenden Bündel, einen eine Blende bildenden Längsspalt (F), der die Erzeugung eines schichtförmigen Beleuchtungsbündels gestattet, das im wesentlichen in einer den Längsspalt (F) enthaltenden Ebene verteilt ist, und Mittel zur Erfassung eines Bündels, das von einem Untersuchungsgegenstand (SU), der mit dem schichtförmigen Beleuchtungsbündel beleuchtet wird, übertragen wird, die eine Gaszelle (10) umfassen, die mit einem Fenster (Fe) für den Eintritt des Beleuchtungsstrahls versehen ist, **dadurch gekennzeichnet, dass** die Erfassungsmittel mindestens einen Detektor (1) für ionisierende Teilchen umfassen, der in dieser Gaszelle mindestens folgendes aufweist:
- eine erste (11), eine zweite (13) und eine dritte (14) ebene Elektrode, wobei jede ebene Elektrode jeweils zu jeder parallel angeordnet ist, um einerseits zwischen der ersten (11) und der zweiten (13) Elektrode einen Raum (12) zur Umwandlung des Beleuchtungsbündels in Elektronen und andererseits zwischen der zweiten (13) und der dritten (14) Elektrode einen Raum (15) zur Verstärkung durch Vermehrung dieser Elektronen zu formen, wobei das Eintrittsfenster (Fe) auf Höhe des Umwandlungsraums angeordnet ist, um den Eintritt des Beleuchtungsstrahls in den Umwandlungsraum zu gewährleisten, wobei der Verstärkungsraum (15) eine Vermehrungslänge, Abstand D zwischen der zweiten (13) und der dritten (14) Elektrode, kleiner als 200 µm besitzt, wobei dieser Detektor außerdem folgendes umfasst:
- Polarisierungsmittel (2), die die erste Elektrode (11) auf ein erstes elektrisches Potential, die zweite Elektrode (13) auf ein zweites elektrisches Potential, das höher als das erste elektrische Potential ist, und die dritte Elektrode (14) auf ein drittes elektrisches Potential bringen können, das höher als das zweite elektrische Potential ist, wobei die zweite Elektrode (13) mit Löchern versehen ist, die eine Kathode bilden, die die Übertragung der im Umwandlungsraum (12) erzeugten Elektronen zu dem Verstärkungsraum (15) gestatten, und die dritten Elektrode (14) eine Anode bildet, die aus einer Vielzahl von elektrisch isolierten Einzelanoden besteht, wobei die an die erste (11), an die zweite (13) und an die dritte Elektrode (14) angelegten elektrischen Potentiale die Erzeugung eines ersten elektrischen Felds E₁ im Umwandlungsraum (12) und eines im wesentlichen parallelen zweiten elektrischen Felds E₂ im Verstärkungsraum (15) gestatten, wobei diese elektrischen Felder E₁, E₂ in einem Amplitudenverhältnis ρ=E₂/E₁ höher als 10 stehen und jedes dieser elektrischen Felder E₁ und E₂ zu diesen Elektroden im wesentlichen rechtwinklig ist, was die Vermehrung dieser Elektroden im Verstärkungsraum durch eine auf die Verstärkungslänge D begrenzte Lawinenerscheinung gestattet, um in Nähe der eine Anode bildenden dritten Elektrode (14) vermehrte Elektronen zu erzeugen,
- Mittel (3) zum Erfassen und Zählen der vervielfachten Elektronen, die mit den die Anode (14) bildenden Einzelanoden gekoppelt sind.

2. Vorrichtung nach Anspruch 1, **dadurch gekennzeichnet, dass** das Eintrittsfenster (Fe) entweder an der Gaszelle (10) seitlich angeordnet ist, so dass es den Eintritt des Beleuchtungsbündels parallel zu den E-lektroden gestattet, oder auf der Seite der Gaszelle (10), die der die Kathodenelektrode bildenden zweiten Elektrode (13) gegenüberliegt, so dass es einen frontalen Eintritt des Beleuchtungsbündels gegenüber den Elektroden gestattet.

3. Vorrichtung nach Anspruch 1 oder 2, **dadurch gekennzeichnet, dass** die Einzelanoden von elektrisch leitenden Elementen (140) mit einer Längssymmetrieachse gebildet sind, wobei die Längssymmetrieachse jedes elektrisch leitenden Elements (140) so ausgerichtet ist, dass sie auf die ionisierende Strahlungsquelle (S), den Punkt der Emission des Beleuchtungsbündels, zu konvergiert, was im wesentlichen die Unterdrückung der Parallaxeabweichung infolge der Projektion der Ebene des Beleuchtungsstrahls auf die Ebene der die Anode bildenden dritten Elektrode (14) gestattet.

4. Vorrichtung nach Anspruch 2 oder 3, **dadurch gekennzeichnet, dass** die die Einzelanoden bildenden elektrisch leitenden Elemente (140) eine Abmessung in einer zu der Richtung der Längssymmetrieachse rechtwinkligen Richtung im wesentlichen gleich dem Verstärkungsabstand (D) aufweisen, wobei zwei in dieser rechtwinkligen Richtung aufeinanderfolgende elektrisch leitende Elemente in dem Öffnungswinkel (Δθ) regelmäßig beabstandet sind.

5. Vorrichtung nach einem der Ansprüche 1 bis 4, **dadurch gekennzeichnet, dass** die Erfassungs- und Zählmittel (3) folgendes umfassen:
- eine Vielzahl von schnellen Verstärkern (30), wobei ein schneller Verstärker mit einem eine Einzelanode (140) bildenden elektrisch leitenden Element gekoppelt ist und ein elektrisches Erfassungssignal für die betreffende Einzelanode (140) liefert,
- am Ausgang der schnellen Verstärker zusammengeschaltete Zähl- und Speichermittel (31).

6. Vorrichtung nach einem der Ansprüche 1 bis 5, **dadurch gekennzeichnet, dass** die zweite Elektrode (13) von einem elektrisch leitenden Gitter gebildet ist und dass das Gitter ein regelmäßiges Netz von Löchern (130i) aufweist, deren Schritt P in der Ebene der zweiten Elektrode zwischen D/10 und D/3 beträgt, wobei die größte Abmessung T der Löcher größer als die Dicke e dieses Gitters, aber kleiner als P ist.

7. Vorrichtung nach einem der Ansprüche 1 bis 6, **dadurch gekennzeichnet, dass** sie außerdem in dem Umwandlungsraum (12) eine Mehrkanalstruktur (121) aufweist, die das Einfangen des Beleuchtungsstrahls und die Erzeugung von Elektronen gestattet, wobei jeder Kanal der Mehrkanalstruktur sich in der Richtung des ersten elektrischen Felds (E₁) erstreckt.

8. Vorrichtung nach Anspruch 7, **dadurch gekennzeichnet, dass** die Mehrkanalstruktur (121) eine Mikrokanalstruktur ist, die von einem Block aus einem die ionisierende Strahlung des Beleuchtungsstrahls absorbierenden Material gebildet wird, wobei diese Struktur mit parallelen Mikrokanälen (121i) versehen ist, die mindestens eine auf einer ebenen Oberfläche dieser Struktur ausmündende Öffnung aufweisen, wobei die Gesamtheit dieser Öffnungen auf dieser ebenen Oberfläche ein Netz von Löchern mit demselben Schritt P und mit derselben Abmessung T wie das Löchernetz in dem die zweite Elektrode (13) bildenden Gitter bildet, diese ebene Oberfläche und dieses Netz von Öffnungen in Nähe dieser zweiten die Kathode bildenden Elektrode (13) angeordnet ist und eine Öffnung dieses Netzes von Öffnungen gegenüber einem Loch (130i) des diese zweite Elektrode bildenden Gitters angeordnet ist, so dass die Öffnungen und die Löcher einander gegenüberstehen.

9. Vorrichtung nach einem der vorhergehenden Ansprüche, **dadurch gekennzeichnet, dass** die Erfassungs- und Zählmittel außerdem eine elektrostatische Erfassungsschaltung aufweisen, die aus einer kapazitiven Kopplung mit jeder Einzelanode (140ᵢ,...,140ₖ...140_{N}) besteht, wobei diese elektrostatische Erfassungsschaltung mindestens ein erstes elektrisch leitendes Band (1410 bis 1430) aufweist, das in einem periodischen Muster in zwei Einzelbänder (1410a, 1410b) unterteilt ist, deren elektrostatische Kopplung mit jeder Einzelanode (140ᵢ, ..., 140ₖ... 140_{N}) eine steigende bzw. abnehmende Funktion der Stelle k der Einzelanode ist, mit der jedes Einzelband gekoppelt ist.

10. Vorrichtung nach einem der Ansprüche 1 bis 9, **dadurch gekennzeichnet, dass** das in der Gaszelle enthaltene Gas mit einem "Quenching"-Dampf gemischtes Xenon mit einem Druck zwischen 10² und 10⁶ Pa ist.

11. Vorrichtung nach Anspruch 1, **dadurch gekennzeichnet, dass** das in der Gaszelle enthaltene Gas aus einer Mischung aus Argon und Trimethylamin besteht, die es gestattet, in dem Verstärkungsraum eine Vermehrung der Elektronen durch Lichtlawinenerscheinung zu bewirken, wobei die Einzelanoden (140) von transparenten elektrisch leitenden Elementen gebildet sind, die die Übertragung des Lichts, das durch die auf diese Weise geschaffenen Lichtlawinen erzeugt wurde, gestattet.

12. Vorrichtung nach Anspruch 11, **dadurch gekennzeichnet, dass** die Einzelanoden (140) von einem elektrisch leitenden Gitter gebildet sind, dem eine Wand zugeordnet ist, die entweder die Übertragung des Lichts mit der Wellenlänge der Lichtlawinenerscheinung oder die Änderung der Wellenlänge der Lichtlawinenerscheinung in eine Wellenlänge des Spektrums des sichtbaren Bereichs gestattet.

13. Vorrichtung nach Anspruch 12, **dadurch gekennzeichnet, dass** die Zählmittel aus Integriermitteln, wie einem empfindlichen Film, bestehen, dem optische Mittel (300a) und Helligkeitsverstärker (300b) zugeordnet sind, die auf der Wand angeordnet sind, die die Übertragung oder die Änderung der Wellenlänge der Lichtlawinenerscheinung gestatten.

14. Vorrichtung nach Anspruch 1 oder 2, **dadurch gekennzeichnet, dass** bei einer radiographischen Bilderzeugung, wobei die ionisierende Strahlungsquelle aus einem Elektrophoresegel oder einem Schnitt besteht, die radioaktiv markiert sind, Sitz von Betastrahlenemission sind und in Nähe des Fensters für einen frontalen Eintritt des Beleuchtungsbündels angeordnet sind, die erste (11) und die zweite (13) Elektrode in einem Abstand X angeordnet sind, der gleich oder kleiner als der Abstand zwischen der zweiten (13) und der dritten Elektrode (14) ist, wobei der Umwandlungsraum und der Verstärkungsraum eine im wesentlichen gleiche Abmessung in einer zu den Elektroden (11, 13, 14) senkrechten Richtung aufweisen, was es gestattet, die Länge des Wegs jedes von der Quelle emittierten Betastrahls auf einen Wert zu begrenzen, der im wesentlichen gleich dem des Abstands X der ersten Elektrode (11) von der zweiten Elektrode (13) ist, und die Genauigkeit der Ortung der Betastrahlenemissionspunkte auf einen Wert zu bringen, der im wesentlichen gleich oder kleiner als dieser Abstand X ist.

15. Hochauflösende Vorrichtung zur radiographischen Bilderzeugung mit ionisierender Strahlung, die eine ionisierende Strahlungsquelle mit einem divergierenden Bündel besitzt, **dadurch gekennzeichnet, dass** sie folgendes aufweist:
- einen Kollimator (CO), der eine Vielzahl von Längsspalten (Fₗ bis Fₙ) besitzt, die eine Vielzahl von Blenden bilden, die die Erzeugung von einer Vielzahl von schichtförmigen Beleuchtungsbündeln gestatten, deren jedes im wesentlichen in einer Ebene verteilt ist, die einen der Längsspalte dieser Vielzahl von Längsspalten enthält, wobei die Gesamtheit der Ebenen, die eines der schichtförmigen Beleuchtungsbündel enthalten, ein Bündel von auf diese ionisierende Strahlungsquelle (S) zu konvergierenden Ebenen bildet, und
- Mittel zur Erfassung der Gesamtheit der schichtförmigen Beleuchtungsbündel, die einen Detektor (lₗ bis lₙ) für ionisierende Teilchen gemäß einem der Ansprüche 1 bis 13 aufweisen, der gegenüber jedem schichtförmigen Beleuchtungsbündel angeordnet ist, wobei jeder Detektor in der Ebene angeordnet ist, die von der ionisierenden Strahlungsquelle und dem schichtförmig liefernden Spalt, dem dieser Detektor zugeordnet ist, gebildet wird.

## Claims

1. A high resolution radiographic imaging device using ionizing radiation comprising a source (S) of ionizing radiation in the form of a divergent beam, a longitudinal slot (F) forming a diaphragm adaptated to deliver a flat illumination beam, substantially distributed in a plane containing the longitudinal slot (F), and means for detecting a beam transmitted through a subject (SU) to be observed, illuminated by the flat illumination beam, comprising one gas chamber (10) provided with an inlet window (Fe) for said illumination beam, wherein said detecting means comprise at least one ionizing particles detector (1) comprising at least:
- a first (11), a second (13) and a third (14) flat electrodes, each flat electrode being placed parallel with one another in order to form in the one hand between the first (11) and second (13) electrodes, a space for the conversion (12) of the illumination beam into electrons and in the other hand, between the second (13) and third (14) electrodes, a space for the amplification (15) by multiplication of these electrons, said inlet window (Fe) being placed at the level of the conversion space in order to ensure the inlet of said illumination beam into said conversion space, said amplification space having a multiplication length D which is formed by the distance separating said second (13) and said third (14) electrodes, of less than 200 µm,
said detector furthermore comprising:
- biasing means (2) adapted to raise said first electrode (11) to a first electrical potential, said second electrode (13) to a second electrical potential higher than said first electrical potential, and said third electrode (14) to a third electrical potential higher than said second electrical potential, said second electrode (13) being pierced with holes and forming a cathode allowing the transmission of the electrons generated in said conversion space (12) towards the amplification space (15), and said third electrode (14) forming an anode constituted by a plurality of electrically isolated elementary anodes, the electrical potentials applied to the first (11), to the second (13) and to the third (14) electrodes allowing the generation of a first E₁ electrical field in the conversion space (15) and a substantially parallel second E₂ electrical field in the amplification space (15), these electrical fields E₁, E₂ having an amplitude ratio ρ=E₂/E₁ greater than 10, each of these electrical fields E₁, E₂ being substantially perpendicular to the said electrodes, thereby allowing the multiplication of these electrons in the amplification space by an avalanche phenomenon confined to said amplification length D in order to generate multiplied electrons in the vicinity of said third electrode (14) forming an anode,
- means (3) for detecting and counting the multiplied electrons which are coupled to said elementary anodes forming said anode (14).

2. The device according to Claim 1, wherein said inlet window (Fe) is placed, either laterally on the gas chamber (10) in order to allow the inlet of the said illumination beam parallel with said electrodes, or on the face of the gas chamber (10) located opposite the second electrode (13) forming the cathode electrode in such a way as to allow frontal inlet of the said illumination beam with respect to said electrodes.

3. The device according to Claim 1 or claim 2, wherein said elementary anodes are formed by electrically conductive elements (140) having an axis of longitudinal symmetry, said axis of longitudinal symmetry of each electrically conductive element (140) being oriented such that they converge towards the ionizing radiation source (S) which forms the point of emission of said illumination beam, thereby allowing to substantially eliminate the parallax error due to the projection of the plane of the illumination beam on the plane of the third electrode (14) constituting said anode.

4. The device according to Claim 2 or claim 3, wherein said electrically conductive elements (140) constituting these elementary anodes have a dimension in a direction perpendicular to said direction of the axis of longitudinal symmetry substantially equal to said amplification distance D, two consecutive electrically conductive elements along this same perpendicular direction being spaced regularly within the angle of divergence (Δθ) of the illumination beam.

5. The device according to Claims 1 to 4, wherein said means (3) of detection and counting comprise:
- a plurality of fast amplifiers (30), one fast amplifier being coupled to an electrically conductive element forming an elementary anode (140), and delivering an electrical detection signal for the elementary anode (140) in question;
- means (31) of counting and storing interconnected at the output of the fast amplifiers.

6. The device according to Claims 1 to 5, wherein said second electrode (13) is formed by an electrically conductive grid, said grid comprising a regular array of holes (130i) whose pitch, P, in the plane of the second electrode is between D/10, D/3, the biggest dimension of the holes T being greater than the thickness e of this grid but less than P.

7. The device according to Claims 1 to 6, further comprising, in said conversion space (12), a multi-channel structure (121) allowing the capture of the illumination beam and the creation of electrons, each channel in said multichannel structure extending in the direction of said first electrical field E₁.

8. The device according to Claim 7, wherein said multi-channel structure (121) is a micro-channels structure formed by a block of material absorbing the ionizing radiation of said illumination beam, said structure being provided with parallel micro-channels (121i) having at least one opening emerging on a flat surface of this structure, the assembly of these openings forming on this flat surface an array of holes at the same pitch P and with the same dimension T as the array of holes in the grid forming said second electrode (13), said flat surface and said array of openings being placed in the vicinity of said second electrode (13) forming said cathode, an opening of this array of openings being placed opposite a hole (130i) of the grid forming said second electrode, the openings and the holes being thus facing one another.

9. The device according to any one of the preceding Claims, wherein said means of detection and counting furthermore comprise an electrostatic detection circuit consisting of a capacitive coupling with each elementary anode (140ₗ, ... 140ₖ, ... 140_{N}), said electrostatic detection circuit comprising at least a first electrically conductive strip (1410 to 1430), said electrically conductive strip being subdivided according to a periodic pattern into two elementary strips (1410a,1410b) whose electrostatic coupling with each elementary anode (140ₗ, ... 140ₖ, ... 140_{N}) are an increasing and decreasing function respectively of the row k of the elementary anode to which each elementary strip is coupled.

10. The device according to any one of the preceding Claims, wherein the gas contained in said gas chamber is xenon mixed with a "quenching" vapor at a pressure between 10² and 10⁶ Pa.

11. The device according to Claim 1, wherein said gas contained in the gas chamber is constituted by a mixture of argon and trimethylamine, so as to allow to cause, in said amplification space, a multiplication of the electrons by the luminous avalanches phenomenon, said elementary anodes (140) being formed by transparent electrically conductive elements, allowing the transmission of the light generated by the luminous avalanches thus created.

12. The device according to Claim 11, wherein said elementary anodes (140) are formed by an electrically conductive grid associated with a surface allowing either the transmission of the light at the wavelength of the luminous avalanche phenomenon, or the changing of the wavelength of the luminous avalanche phenomenon into a wavelength in the visible range spectrum.

13. The device according to Claim 12, wherein said means of counting are constituted by integrating means, such as a sensitive film, associated with optical (300a) and brilliance amplifier (300b) means, placed on the surface allowing the transmission or the change in wavelength of the luminous avalanche phenomenon.

14. The device according to Claim 1 or 2, wherein for imaging using β radiography, the ionizing radiation source consisting of an electrophoresis gel or a radioactively marked section which is the site of β ray emission points and placed in the vicinity of the window for a frontal inlet of the illumination beam, said first (11) and second (13) electrodes are distant by a distance X equal to or less than the distance separating said second (13) and said third (14) electrodes, said conversion space and said amplification space having a dimension substantially equal in a direction perpendicular to the said electrodes (11,13,14), thereby allowing to limit the length of the path of each β ray emitted by the source to a value substantially equal to that of the distance X separating the first (11) and the second (13) electrodes and to bring an accuracy of location of the β ray emission points to a value close to or less than this distance X.

15. A high resolution radiographic imaging device using ionizing radiation, comprising a source of ionizing radiation in the form of a divergent beam, said device including:
- a collimator (CO) comprising a plurality of longitudinal slots (Fₗ to Fₙ) forming a plurality of diaphragms adapted to deliver a plurality of flat illumination beams, each one distributed substantially in a plane containing one of the longitudinal slots of this plurality of longitudinal slots, the planes containing one of the flat illumination beams together forming a beam of planes converging towards said ionizing radiation source S, and
- means for detecting the set of flat illumination beams, said means of detection including at least one ionizing particles detector (lₗ to lₙ) according to any one of claims 1 to 13, placed facing each flat illumination beam, each detector being placed in the plane formed by the ionizing radiation source and the slot delivering the flat illumination beam with which this detector is associated.
